# EUROPÄISCHE PATENTSCHRIFT

(11) **EP 1 317 430 B1**
(45) Veröffentlichungstag und Bekanntmachung des Hinweises auf die Patenterteilung: **18.05.2005**
(21) Anmeldenummer: 01965274.2
(22) Anmeldetag: 11.09.2001
(51) Int. Cl.: C07D 209/52, C07D 401/12, A61K 31/403, A61P 25/00

(54) **(2-AZABICYCLO [2.2.1]HEPT-7-YL)METHANOL-DERIVATE ALS NIKOTINISCHE ACETYLCHOLINREZEPTOR AGONISTEN**
(2-AZABICYCLO [2.2.1]HEPT-7-YL)METHANOL DERIVATIVES AS NICOTINIC ACETYLCHOLINE RECEPTOR AGONISTS
DERIVES DE (2-AZABICYCLO [2.2.1]HEPT-7-YL)METHANOL UTILISES COMME ANGONISTES DU RECEPTEUR DE L'ACETYLCHOLINE NICOTINIQUE

(30) Priorität: 12.09.2000 DE 10044905
(43) Veröffentlichungstag der Anmeldung: 11.06.2003
(73) Patentinhaber: MERCK PATENT GmbH, 64293 Darmstadt (DE)
(72) Erfinder: SCHIEMANN, Kai, 64297 Darmstadt (DE); LEIBROCK, Joachim, 64319 Pfungstadt (DE)
(74) Vertreter: Isenbruck, Günter, Dr.
(86) Internationale Anmeldenummer: PCT/EP2001/010491
(87) Internationale Veröffentlichungsnummer: WO 2002/022578

(56) Entgegenhaltungen:
- EP-A- 0 978 280
- WO-A-00/23424
- WO-A-92/05172
- US-A- 5 817 679

## Beschreibung

Die vorliegende Erfindung betrifft Substanzen, die zur Behandlung von Krankheiten eingesetzt werden, bei denen die Anregung der nikotinischen Acetylcholinrezeptoren zur Verbesserung des Krankheitsbildes führt. Die erfindungsgemäßen Substanzen enthalten eine gegebenenfalls substituierte (2-Azabicyclo[2.2.1]hept-7-yl-methyl)-Einheit, die mit einer Carbamat-, Thiocarbamat- oder Hamstoffeinheit verbunden ist.

Von der gut charakterisierten Klasse der Acetylcholinrezeptoren werden einige Mitglieder für bestimmte Krankheitsbilder des zentralen Nervensystems verantwortlich gemacht. Bekannte Wirkstoffe, die mit der Klasse der Acetylcholinrezeptoren wechselwirken können, sind beispielsweise Pilocarpin, Nicotin, Lobelin und Epibatidin.

Es besteht jedoch weiterhin ein Bedarf an Verbindungen, die zur Behandlung von Krankheitsbildern eingesetzt werden können, die durch eine Dysfunktion nikotinischer Acetylcholinrezeptoren hervorgerufen werden.

Die Aufgabe der vorliegenden Erfindung ist es, Verbindungen zur Verfügung zu stellen, mit denen diese Krankheitsbilder behandelt werden können. Diese Aufgabe wird gelöst durch Substanzen der allgemeinen Formel (I) 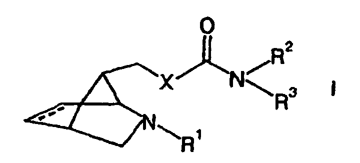 in der
die gestrichelte Linie für eine optional vorhandene Doppelbindung steht, X ausgewählt ist aus der Gruppe bestehend aus NH, NR⁴, O und S,
R¹ ausgewählt ist aus der Gruppe bestehend aus Wasserstoff, linearen und verzweigten, substituierten und unsubstituierten C₁-C₁₀-Alkylgruppen, substituierten und unsubstituierten C₅-C₁₀-Arylgruppen, substituierten und unsubstituierten C₄-C₁₀-Heteroaryl-Gruppen, Acylgruppen, Thioacylgruppen, Carbonylcarboxygruppen, N-organylsubstituierten Carbamoylgruppen und Organosulfonylgruppen,
R², R³ und R⁴ unabhängig voneinander ausgewählt sind aus der Gruppe bestehend aus C₁-C₁₀-Alkylgruppen, C₅-C₁₂-Arylgruppen und C₄-C₁₂-Heteroarylgruppen, wobei diese Gruppen unsubstituiert sind oder einen oder mehrere Substituenten aufweisen, die unabhängig voneinander ausgewählt sind aus der Gruppe bestehend aus Halogenen, Alkoxygruppen, Aryloxygruppen, Nitrogruppen, C₁-C₅-Alkoxycarbonylgruppen, gegebenenfalls durch Fluor substituierten Alkyl- oder Acylgruppen und Cyanogruppen.

Es wurde gefunden, daß die Substanzen gemäß der Formel (I) spezifisch zur Behandlung von Krankheiten eingesetzt werden können, bei denen die Anregung der nikotinischen Acetylcholinrezeptoren zur Verbesserung des Krankheitsbilds führt. Beispiele sind dem Fachmann bekannt und umfassen Schizophrenie, Demenz, dabei auch insbesondere Morbus Alzheimer, neurodegenerative Erkrankungen, Parkinsonsche Krankheit und Tourette's Syndrom. Ebenso agieren die Substanzen neuroprotektiv, was den Einsatz bei Schlaganfall oder Vergiftungen ermöglichen sollte.

Gemäß einer Ausführungsform der vorliegenden Erfindung ist es bevorzugt, wenn in den Substanzen der Formel (I) X = NH, NR⁴ oder O, insbesondere O ist,

R¹ ausgewählt ist aus der Gruppe bestehend aus Wasserstoff, linearen und verzweigten C₁-C₅-Alkylgruppen, die unsubstituiert sind oder als Substituenten einen oder mehrere Halogenatome, gegebenenfalls alkylsubstituierte C₆-C₁₀-Arylgruppen und/oder C₁-C₅-Alkoxycarbonylgruppen tragen können, C₆-C₁₂-Arylgruppen und C₅-C₁₀-Heteroarylgruppen, die unsubstituiert sein oder als Substituenten jeweils ein oder mehrere Halogenatome und/oder Alkylgruppen tragen können, aliphatischen C₁-C₅-Acyl- und Thioacylgruppen, aromatischen C₆-C₉-Aroyl- und Thioaroylgruppen, Carbamoylgruppen des Typs R⁵-N(H)-C(O)- und Sulfonylgruppen des Typs R⁵-SO₂-,
R², R³ und R⁴ unabhängig voneinander ausgewählt sind aus der Gruppe bestehend aus C₁-C₄-Alkylgruppen, C₅-C₉-Arylgruppen und C₅-C₈-Heteroarylgruppen, die unsubstituiert sind oder einen oder mehrere Substituenten tragen, die ausgewählt sind aus der Gruppe bestehend aus C₁-C₄-Alkoxygruppen, C₆-C₉-Aryloxygruppen, Nitrogruppen, C₁-C₂-Alkoxycarbonylgruppen, Cyanogruppen, Fluorid, Chlorid, Fluormethylgruppen und Acetylgruppen.

R⁵ ausgewählt ist aus der Gruppe bestehend aus C₁-C₃-Alkylgruppen, C₁-C₃-Fluoralkylgrupen und Phenylgruppen, die unsubstituiert oder durch einen oder mehrere Methyl- oder Trifluormethylgruppen substituiert sein können.

Gemäß einer weiteren Ausführungsform der vorliegenden Erfindung ist es bevorzugt, wenn die Verbindungen der Formel (I) keine Doppelbindung aufweisen.

Die Herstellung der Verbindungen der Formel (I) erfolgt generell nach an sich bekannten Methoden, wie sie in der Literatur (z.B. J. March, Advanced Organic Chemistry, 3^{rd} edition, John Wiley & Sons, New York oder Houben-Weyl, Methoden der Organischen Chemie, Georg-Thieme-Verlag, Stuttgart) beschrieben sind, und zwar unter Reaktionsbedingungen, wie sie für die genannten Umsetzungen bekannt und geeignet sind. Dabei kann man auch auf an sich bekannte, nachfolgend nicht näher erwähnte Varianten zurückgreifen.

Gemäß der vorliegenden Erfindung werden die Substanzen der Formel (I) vorzugsweise hergestellt, indem eine Substanz der Formel (II), die sich von der Formel (I) durch den Ersatz einer Gruppe R¹ gegen ein Wasserstoffatom ableitet, mit einem von der Gruppe R¹ abgeleiteten, ein geeignetes Gegenion aufweisendes Kation R¹⁺ umgesetzt wird. Dies ist in der nachfolgenden Gleichung (1) dargestellt.

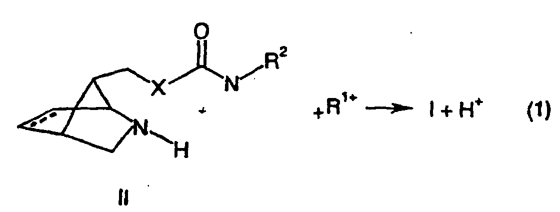

In der Gleichung (1) haben die Substituenten R¹ und R² die für die Formel (I) angegebene Bedeutung. Die Verbindungen der Formel (II) werden aus den Verbindungen der Formel (III), in denen A eine geeignete Schutzgruppe für die Aminofunktion ist, hergestellt.

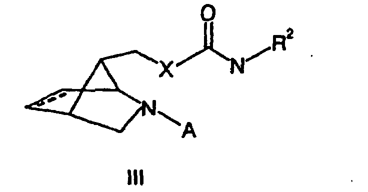

Vorzugsweise wird eine Benzylgruppe als Schutzgruppe verwendet. Aus diesen Verbindungen (III) sind die erfindungsgemäßen Substanzen durch Abspaltung der Schutzgruppe herstellbar, was vorzugsweise durch Hydrierung, beispielsweise mittels Palladium auf Aktivkohle, geschieht.

Die Substanzen der Formel (III) wiederum sind aus den Verbindungen entsprechend Formel (IV) erhältlich, und zwar durch Reaktion mit dem entsprechenden, den gewünschten Rest R² aufweisenden lsocyanat R²NCO

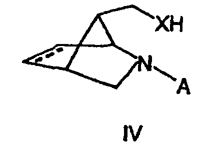

Die Verbindungen (IV) sind im Fall von X = O bekannt, siehe beispielsweise WO 92/05172 und darin zitierte Literatur. Die Verbindungen (IV), in denen X = S ist, können nach literaturbekannten Verfahren aus den entsprechenden Verbindungen (IV) mit X = O hergestellt werden.

Im Fall der Verbindungen (IV) mit X = NH oder NR⁴ können diese aus den entsprechenden Alkoholen (X = O) nach verschiedenen Arten erhalten werden. Beispielsweise wird der entsprechende Alkohol mit einem Sulfonylchlorid, beispielsweise Toluolsulfonylchlorid, umgesetzt und der erhaltene Sulfonsäurerest dann mit einem Azid umgesetzt. Derart erhält man das entsprechende Azid des jeweiligen Aza-bicyclo[2.2.1]heptans. Dieser Azid läßt sich mit den üblichen Reduktionsmitteln, beispielsweise LiAlH₄, zu dem entsprechenden primären Amin reduzieren, wobei eine Verbindung (IV) mit X = NH erhalten wird.

Das primäre Amin ist auch direkt aus dem erwähnten Alkohol in einer komplexen Reaktion zugänglich. In dieser wird der Alkohol mit Stickstoffwasserstoffsäure HN₃ und einem Azodicarboxylat in Gegenwart eines geeigneten Phosphins, beispielsweise Triphenylphosphin, zur Reaktion gebracht.

Das primäre Amin läßt sich auch in das sekundäre Amin überführen, das einen Substituenten R⁴ aufweist, beispielsweise durch Acylierung und Reduktion der Carbonylfunktion, wobei die gewünschte Verbindung (IV) mit X = NR⁴ erhalten wird.

Die Verbindungen der Formel (I) weisen zumindest ein asymmetrisches Kohlenstoffatom auf, das unterschiedliche Konfigurationen aufweisen kann. Sie können daher in verschiedenen optisch aktiven Formen oder auch als Racemate bzw. Racematgemisch vorliegen.

Eine Base der Formel (I) kann mit einer Säure in das zugehörige Säureadditionssalz übergeführt werden. Für diese Umsetzung eignen sich bevorzugt Säuren, die physiologisch unbedenkliche Salze liefern. So können anorganische Säuren verwendet werden, beispielsweise Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure. Ebenfalls eignen sich organische Säuren, beispielsweise aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- und Ethansulfonsäure, Benzolsulfonsäure, p-Toluolsulfonsäure, Napthalin-mono- und -disulfonsäuren, Laurylschwefelsäure. Säureadditionssalze, die nicht physiologisch unbedenklich sind (Pikrate), können sich zur Isolierung und Aufreinigung der Basen der Formel (I) eignen.

Eine Base der Formel (I) kann bei einigen Verbindungen (beispielsweise im Fall von X = NH) aus einem ihrer Salze mit starken Basen wie Natrium- oder Kaliumhydroxid, Natrium- oder Kaliumcarbonat in Freiheit gesetzt werden.

Die oben dargelegten Verbindungen (I) werden zur Herstellung von Arzneimitteln verwendet, bei denen die Anregung der nikotinischen Acetylcholinrezeptoren das Krankheitsbild positiv verändert.

Diese nikotinischen Acetylcholinrezeptoren lassen sich in zwei prinzipielle Hauptklassen unterteilen, in Abhängigkeit von den Orten, an denen sie vorkommen.

Zum einen sind dies die neuromuskulären Rezeptoren. Diese werden weiter unterteilt in (α₁α₁βεδ) ― und (α₁α₁βγδ) ― Rezeptoren. Zum anderen existieren die neuronalen nikotinischen Acetylcholinrezeptoren, die in den Ganglien gefunden werden. Bei diesen unterscheidet man zwischen den (β₂-β₅) - Rezeptoren und den (α₂-α₉) - Rezeptoren, siehe hierzu auch "Basic Neurochemistry", Ed. Siegel et. al., Raven Press, New York 1993.

Die Substanzen der Formel (I) sind in der Lage, mehr oder weniger gut, etwa in Abhängigkeit von der Struktur des jeweils eingesetzten Moleküls, mit jedem dieser Rezeptoren eine Wechselwirkung einzugehen. Besonders gut wechselwirken die Substanzen der Formel (I), dabei insbesondere die nachstehend als bevorzugt beschriebenen, mit dem nikotinischen α₇-Rezeptor.

Ein in-vitro Nachweis der Wechselwirkungen mit dem nikotinischen α₇-Rezeptor kann beispielsweise analog zu J.M. Ward et al., FEBS 1990, 270, 45-48 oder D.R.E. Macallan, FEB 1998, 226, 357-363, erfolgen. Weitere in-vitro Tests für nikotinische Rezeptoren sind in F.E. D'Amour et al., Manual for Laboratory Work in Mammalian Physiology, 3^{rd} Ed., The U-niversity of Chicago Press (1965), W. Sihver et al., Neuroscience 1998, 85, 1121-1133 oder B. Latli et al., J.Med. Chem. 1999, 42, 2227-2234, beschrieben.

Krankheiten, die mit den Substanzen gemäß Formel (I) behandelt werden können, umfassen Schizophrenie, Demenz, dabei insbesondere Morbus Alzheimer, neurodegenerative Erkrankungen, Parkinson'sche Krankheit, Tourette's Syndrom, altersbedingte Gedächtnisschwäche, Linderung von Entzugserscheinungen, außerdem durch die neuroprotektive Wirkung Anwendung bei Schlaganfall und Schädigung des Gehirns durch toxische Verbindungen.

Ein weiterer Gegenstand der vorliegenden Erfindung sind pharmazeutische Zubereitungen enthaltend eine oder mehrere Verbindungen entsprechend der Formel (I) und/oder deren physiologisch wirksame Salze. Dazu können diese zusammen mit mindestens einem Träger- oder Hilfsstoff und gegebenenfalls in Kombination mit einem oder mehreren weiteren Wirkstoffen in eine geeignete Darreichungsform gebracht werden. Diese Zubereitungen können als Arzneimittel in der Human- und Veterinärmedizin eingesetzt werden. Als Trägersubstanzen kommen organische oder anorganische Stoffe in Frage, die sich für die enterale (beispielsweise orale), parenterale oder topische Applikation eignen und mit den neuen Verbindungen nicht reagieren. Beispiele umfassen Wasser, pflanzliche Öle, Benzylalkohole, Polyethylenglykole, Gelatine, Kohlenhydrate wie Lactose oder Stärke, Magnesiumstearat, Talk und Vaseline. Zur enteralen Applikation dienen insbesondere Tabletten, Dragees, Kapseln, Sirupe, Säfte, Tropfen oder Suppositorien, zur patenteralen Applikation Lösungen, vorzugsweise ölige oder wäßrige Lösungen, ferner Suspensionen, Emulsionen oder Implantate, für die topische Anwendungen, Salben, Cremes, Pflaster oder Puder. Die neuen Verbindungen können auch lyophilisiert und die erhaltenen Lyophilisate beispielsweise zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können gegebenenfalls auch einen oder mehrere weitere Wirkstoffe enthalten, die nicht der Formel (I) entsprechen, beispielsweise ein oder mehrere Vitamine.

Dabei werden die erfindungsgemäßen Substanzen in der Regel in Analogie zu bekannten, im Handel befindlichen Präparaten (beispielsweise Tacrin) verabreicht, vorzugsweise in Dosierungen zwischen etwa 5 mg und 100 mg, insbesondere zwischen 10 und 40 mg pro Dosierungseinheit. Die tägliche Dosierung liegt vorzugsweise zwischen etwa 0,5 und 1 mg/kg Körpergewicht.

Die spezielle Dosis für jeden einzelnen Patienten hängt von verschiedensten Faktoren ab, beispielsweise von der Wirksamkeit der eingesetzten speziellen Verbindung, vom Alter, Körpergewicht, allgemeinem Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt.

Die orale Anwendung ist bevorzugt.

Die Erfindung wird nun in den nachfolgenden Beispielen erläutert:

### Beispiele:

### Beispiel 1:

200,00 mg (0,920 mmol) (2-Benzyl-2-aza-bicyclo[2.2.1]hept-7yl)-methanol wurde in 3 ml trockenem THF gelöst und anschließend 176,00 mg (0,920 mmol) Ethyl-3-isocyanatobenzoat in 1 ml trockenem THF bei Raumtemperatur zugegeben. Nach Rühren über 6 Stunden bei Raumtemperatur wurden 125 mg (0,30 mmol) Tris-(2-aminoethyl)-amin-Polystyrol und 250 mg (0,30 mmol) Methylisocyanat-Polystyrol (jeweils NovaBiochem) zugefügt und bei 50°C über Nacht gerührt. Nach dem Abfiltrieren der Harze wurde das Lösungsmittel entfernt und das gewünschte Produkt chromatographisch mit Essigester als Laufmittel aufgereinigt. Auf diese Weise wurden 220,00 mg (0,539 mmol) 3-(2-Benzyl-2-aza-bicyclo[2.2.1]hept-7-ylmethoxycarbonylamino)-benzoesäure-ethylester in Form eines farblosen Feststoffs erhalten.

150,00 mg (0,367 mmol) dieser Verbindung wurde anschließend in 2 ml Methanol aufgenommen und nach Zugabe von 10,00 mg Palladium auf Aktivkohle 18 Stunden unter Wasserstoffatmosphäre gerührt. Nach Abfiltrieren des Katalysators und dem Entfernen des Lösungsmittels wurde ein farbloser Rückstand (103,00 mg, 0,324 mmol) enthaltend 3-(2-Azabicyclo[2.2.1]hept-7-ylmethoxycarbonylamino)-benzoesäure-ethylester erhalten.

15,919 mg (0,050 mmol) dieser Verbindung wurden in 0,5 ml THF gelöst, anschließend 10,119 mg (0,10 mmol) Triethylamin zugegeben und danach 6,125 mg (0,060 mmol) Acetanhydrid zugetropft. Die Lösung wurde 18 Stunden bei Raumtemperatur gerührt, mit Wasser gequencht und die organische Phase nach Zugabe von Ethylacetat (2 ml) isoliert. Nach Entfernen des Lösungsmittels und Trocknen wurden 18,00 mg roher 3-(2-Acetyl-2-aza-bicyclo[2.2.1]hept-7-yl-methoxycarbonylamino)-benzoesäureethylester in Form eines farblosen Öls erhalten, das nach den üblichen Methoden weiter gereinigt werden kann. M⁺ (EI): 361

Analog wurden erhalten:
(2,6-Dichloro-phenyl)-carbamatsäure-2-benzyl-2-azabicyclo[2.2.1]hept-7-yl-methyl-ester; M⁺ (EI): 406
(4-Phenoxy-phenyl)-carbamatsäure-2-benzyl-2-azabicyclo[2.2.1]hept-7-yl-methyl-ester; M⁺ (EI): 430
(2-Nitro-phenyl)-carbamatsäure-2-benzyl-2-aza-bicyclo[2.2.1]hept-7-yl-methyl-ester; M⁺ (EI): 382
3-(2-Benzyl-2-aza-bicyclo[2.2.1]hept-7-yl-methoxycarbonylamino)-benzoesäure-ethylester; M⁺ (EI): 409
(3,4-Dichloro-phenyl)-carbamatsäure-2-aza-bicyclo[2.2.1]hept-7-yl-methylester; M⁺ (EI): 316
(3,5-Bis-Trifluoromethyl-phenyl)-carbamatsäure-2-aza-bicyclo[2.2.1]hept-7-yl-methyl-ester; M⁺ (EI): 383
(2,4-Dimethoxy-phenyl)-carbamatsäure-2-azabicyclo[2.2.1]hept-7-yl-methyl-ester; M⁺ (EI): 307
3-(2-Aza-bicyclo[2.2.1]hept-7-yl-methoxycarbonylamino)-benzoesäureethylester; M⁺ (EI): 319
(4-Fluoro-3-nitro-phenyl)-carbamatsäure-2-benzyl-2-aza-bicyclo[2.2.1]hept-7-yl-methyl-ester; M⁺ (EI): 400
(3,5-Bis-trifluoromethyl-phenyl)-carbamatsäure-2-benzyl-2-aza-bicyclo[2.2.1]hept-7-yl-methyl-ester; M⁺ (EI): 473
(3,4,5,-Trimethoxy-phenyl)-carbamatsäure-2-benzyl-2-aza-bicyclo[2.2.1]hept-7-yl-methyl-ester; M⁺ (EI): 428
(3,4-Dichloro-phenyl)-carbamatsäure-2-benzyl-2-aza-bicyclo[2.2.1]hept-7-yl-methyl-ester; M⁺ (EI): 406
3-[2-(4-Trifluoromethyl-phenylcarbamoyl)-2-aza-bicyclo[2.2.1]hept-7-yl-methoxycarbonylamino]-benzoesäure-ethylester; M⁺ (EI): 506
3-[2-(Toluene-4-sulfonyl)-2-aza-bicyclo-[2.2.1]hept-7-yl-methoxycarbonylamino]-benzoesäure-ethylester; M⁺ (EI): 474
3-(2-Ethoxycarbonylmethyl-2-aza-bicyclo[2.2.1]hept-7-yl-methoxycarbonylamino)-benzoesäure-ethylester; M⁺ (EI): 405
(3-Cyano-phenyl)-carbamatsäure-2-benzyl-2-aza-bicyclo[2.2.1]hept-7-yl-methyl-ester; M⁺ (EI): 362
(2,6-Dichloro-pyridin-4-yl)-carbamatsäure-2-benzyl-2-aza-bicyclo[2.2.1]hept-7-yl-methyl-ester; M⁺ (EI): 407
(3-Acetyl-phenyl)-carbamatsäure-2-benzyl-2-aza-bicyclo[2.2.1]hept-7-yl-methyl-ester; M⁺ (EI): 379
1-(2-Benzyl-2-aza-bicyclo[2.2.1]hept-7-ylmethyl)-3-(3,4,5-trimethoxy-phenyl)-harnstoff; M⁺ (EI): 427
1-(2-Benzyl-2-aza-bicyclo[2.2.1]hept-7-ylmethyl)-3-(2-nitro-phenyl)-harnstoff; M⁺ (EI): 381.

### Beispiel 2:

1) Zu einer gut gerührten Lösung von 0,6 g Tosylchlorid in 5 ml trockenem Pyridin wurden 0,5 g (2-Benzyl-2-aza-bicyclo[2.2.1]hept-7-yl)-methanol in 2 ml trockenem Pyridin bei 0°C gegeben. Die Mischung wurde über 12 h bei 0°C gerührt, auf Eis gegossen und eine weitere Stunde gerührt. Das erhaltene Öl wurde mit Essigester extrahiert, die organische Phase zweimal mit Wasser gewaschen, dann getrocknet und zur Trockne eingeengt. Das erhaltene (2-Benzyl-2-aza-bicyclo[2.2.1]hept-7-yl-methyl)-toluol-4-sulfonsäureester wurde ohne weitere Reinigung in die Stufe 2) eingesetzt.
   Ausbeute: 99,3 % d. Th. ESI-MS: 372
2) Zu einer Suspension von NaN₃ (0,715 g) in 10 ml Dimethylformamid wurde das in 3 ml Dimethylformamid gelöste, in Schritt 1) erhaltene Produkt (0,583 g) bei Raumtemperatur gegeben. Nach vollendeter Zugabe wurde die Mischung bei 85° über 18 h gerührt, auf Eiswasser gegossen und mit Essigester extrahiert. Die organische Phase wurde getrocknet und unter vermindertem Druck zu einem farblosen Öl konzentriert, bei dem es sich um reines 7-Azidomethyl-2-benzyl-2-aza-bicyclo[2.2.1]heptan handelte.
   Ausbeute: 84,1 % d. Th. ESI-MS: 243
3) Zu einer Suspension von 0,114 g LiAlH₄ in 10 ml THF wurde tropfenweise eine Lösung des in Schritt 2) erhaltenen Produkts (0,320 g) in 5 ml THF gegeben. Nach beendeter Zugabe wurde die Mischung über Nacht bei Raumtemperatur gerührt. Eine 2N NaOH-Lösung wurde zugegeben und die Mischung filtriert, mit Essigester gewaschen, die organische Phase abgetrennt und die wäßrige Phase erneut mit Essigester extrahiert. Die vereinigten organischen Phasen wurden getrocknet und das Lösungsmittel entfernt. Das erhaltene (2-Benzyl-2-aza-bicyclo[2.2.1.]hept-7-yl)-methylamin wurde teilweise in das entsprechende Dihydrochlorid überführt.
   Ausbeute: 80,5 % d. Th. ESI-MS: 217

### Beispiel 3:

Eine 0,4 M Lösung von 0,108 g Stickstoffwasserstoffsäure in 6,25 ml Toluol wurde zu einer Lösung von (2-Benzyl-2-aza-bicyclo[2.2.1]hept-7-yl)-methanol (217 mg) in 1 ml wasserfreiem THF gegeben. Anschließend wurde eine Lösung von 0,461 g Diisopropyl Azodicarboxylat in 1,5 ml THF und eine Lösung von 1,31 g Triphenylphosphin in 2 ml THF zugegeben. Die Temperatur der Lösung wurde durch dosierte Zugabe dieser letzten Lösung bei 25°C gehalten. Die Lösung wurde insgesamt über 19 h bei Temperaturen zwischen 25 und 50°C gerührt, anschließend wurden 0,5 ml Wasser zugegeben, die Mischung bei 50°C über weitere 6 h gerührt, das Lösungsmittel im Vakuum entfernt und der erhaltene Rückstand mit 10 ml Methylenchlorid und 10 ml 1 N HCl versetzt. Die wäßrige Phase wurde abgetrennt und wiederholt mit Methylenchlorid gewaschen. Durch Entfernen des Wassers unter vermindertem Druck wurde (2-Benzyl-2-aza-bicyclo[2.2.1]hept-7-yl)-methylamin in Form eines farblosen Öls erhalten.
Ausbeute: 52 % d. Th.

### Beispiel 4:

Das nach den Beispielen 2 bzw. 3 erhaltene Amin (30 mg) wurde in 1 ml THF gelöst, bei Raumtemperatur 28,131 mg Triethylamin zugegeben und 17,355 mg Essigsäureanhydrid zugetropft. Die Lösung wurde 18 h bei Raumtemperatur gerührt, mit Wasser gequencht und die organische Phase nach Zugabe von Essigester isoliert. Nach Trocknen und Entfernen des Lösungsmittels wurde (2-Benzyl-2-aza-bicyclo-[2.2.1]hept-7-ylmethyl)-acetamid erhalten.
Ausbeute: 89 % d. Th. ESI-MS: 259

### Beispiel 5:

3,795 mg LiAlH₄ wurden in 1 ml THF eingebracht und 20 mg des in Beispiel 4 erhaltene Amids, die in 1 ml THF gelöst waren, zugetropft. Die Mischung wurde 1 h bei 0°C, anschließend bei Raumtemperatur gerührt. Dann wurde die Mischung mit Wasser versetzt, eine wäßrige NH₃-Lösung zugefügt, filtriert und mit Essigester extrahiert. Die organische Phase wurde getrocknet und das Lösungsmittel verdampft. So wurden 18 mg (2-Benzyl-2-aza-bicyclo-[2.2.1]hept-7-ylmethyl)ethylamin erhalten.
Ausbeute: 96 % d. Th. ESI-MS: 245

Ebenfalls wurde (2-Benzyl-2-aza-bicyclo-[2.2.1]hept-7-yl)-methandiol hergestellt, ESI-MS: 234

Die nachstehenden Beispiele betreffen pharmazeutische Zubereitungen, die Substanzen der Formel I oder eines ihrer Säureadditionssalze enthalten:

### Beispiel A: Tabletten

Das Gemisch von 1 kg 3-(2-Acetyl-2-aza-bicyclo[2.2.1]hept-7-yl-methoxycarbonylamino)-benzoesäure-ethylester 4 kg Lactose, 1,2 kg Kartoffelstärke, 0,2 kg Talk und 0,1 kg Magnesiumstearat wird in üblicher Weise zu Tabletten verpreßt, so daß jede Tablette 10 mg Wirkstoff enthält.

### Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

### Beispiel C: Kapseln

2 kg 3-(2-Acetyl-2-aza-bicyclo[2.2.1]hept-7-yl-methoxycarbonylamino)-benzoesäure-ethylester werden in üblicher Weise in Hartgelatinekapseln gefüllt, so daß jede Kapsel 20 mg Wirkstoff enthält.

### Beispiel D: Ampullen

Eine Lösung von 1 kg 3-(2-Acetyl-2-aza-bicyclo[2.2.1]hept-7-yl-methoxycarbonylamino)-benzoesäure-ethylester in 601 zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten Dragees, Kapseln und Ampullen erhältlich, die eine andere Verbindung der Formel I und/oder ein oder mehrere physiologisch unbedenkliche Säureadditionssalze einer Verbindung der Formel (I) enthalten.

## Patentansprüche

1. Substanz der allgemeinen Formel 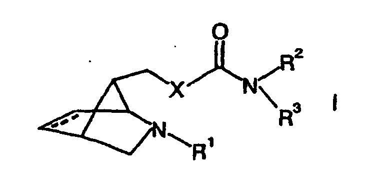 in der
die gestrichelte Linie für eine optional vorhandene Doppelbindung steht,
X ausgewählt ist aus der Gruppe bestehend aus NH, NR⁴, O und S,
R¹ ausgewählt ist aus der Gruppe bestehend aus Wasserstoff, linearen und verzweigten, substituierten und unsubstituierten C₁-C₁₀-Alkylgruppen, substituierten und unsubstituierten C₅-C₁₀-Arylgruppen, substituierten und unsubstituierten C₄-C₁₀-Heteroaryl-Gruppen, Acylgruppen, Thioacylgruppen, Carbonylcarboxygruppen, N-organylsubstituierten Carbamoylgruppen und Organosulfonylgruppen,
R², R³ und R⁴ unabhängig voneinander ausgewählt sind aus der Gruppe bestehend aus C₁-C₁₀-Alkylgruppen, C₅-C₁₂-Arylgrupen und C₄-C₁₂-Heteroarylgruppen, wobei diese Gruppen unsubstituiert sind oder einen oder mehrere Substituenten aufweisen, die unabhängig voneinander ausgewählt sind aus der Gruppe bestehend aus Halogenen, Alkoxygruppen, Aryloxygruppen, Nitrogrupen, C₁-C₅-Alkoxycarbonylgruppen, gegebenenfalls durch Fluor substituierten Alkyl- oder Acylgruppen und Cyanogruppen,
oder ein physiologisch verträgliches Salz davon.

2. Substanz nach Anspruch 1, in denen X = NH, NR⁴ oder O, insbesondere O ist,
R¹ ausgewählt ist aus der Gruppe bestehend aus Wasserstoff, linearen und verzweigten C₁-C₅-Alkylgruppen, die unsubstituiert sind oder als Substituenten einen oder mehrere Halogenatome, gegebenenfalls alkylsubstituierte C₆-C₁₀-Arylgruppen und/oder C₁-C₅-Alkoxycarbonylgruppen tragen können, C₆-C₁₂-Arylgruppen und C₅-C₁₀-Heteroarylgruppen, die unsubstituiert sein oder als Substituenten jeweils ein oder mehrere Halogenatome und/oder Alkylgrupen tragen können, aliphatischen C₁-C₅-Acyl- und Thioacylgruppen, aromatischen C₆-C₉-Aroyl- und Thioaroylgruppen, Carbamoylgruppen des Typs R⁵-N(H)-C(O)- und Sulfonylgruppen des Typs R⁵-SO₂-,
R², R³ und R⁴ unabhängig voneinander ausgewählt sind aus der Gruppe bestehend aus C₁-C₄-Alkylgruppen, die C₅-C₉-Arylgruppen und C₅-C₈-Heteroarylgruppen, die unsubstituiert sind oder einen oder mehrere Substituenten tragen, die ausgewählt sind aus der Gruppe bestehend aus C₁-C₄-Alkoxygruppen, C₆-C₉-Aryloxygruppen, Nitrogruppen, C₁-C₂-Alkoxycarbonylgruppen, Cyanogruppen, Fluorid, Chlorid, Fluormethylgruppen und Acetylgruppen,
R⁵ ausgewählt ist aus der Gruppe bestehend aus C₁-C₃-Alkylgruppen, C₁-C₃-Fluoralkylgruppen und Phenylgruppen, die unsubstituiert oder durch einen oder mehrere Methyl- oder Trifluormethylgruppen substituiert sein können, oder ein physiologisch verträgliches Salz davon.

3. (2,6-Dichloro-phenyl)-carbamatsäure-2-benzyl-2-azabicyclo[2.2.1] hept-7-yl-methyl-ester,
(4-Phenoxy-phenyl)-carbamatsäure-2-benzyl-2-azabicyclo[2.2.1]hept-7-yl-methyl-ester,
(2-Nitro-phenyl)-carbamatsäure-2-benzyl-2-aza-bicyclo[2.2.1] hept-7-yl-methyl-ester,
3-(2-Benzyl-2-aza-bicyclo[2.2.1]hept-7-yl-methoxycarbonylamino)-benzoesäure-ethylester,
(3,4-Dichloro-phenyl)-carbamatsäure-2-aza-bicyclo[2.2.1] hept-7-yl-methyl-ester,
(3,5-Bis-Trifluoromethyl-phenyl)-carbamatsäure-2-aza-bicyclo[2.2.1 ]hept-7-yl-methyl-ester,
(2,4-Dimethoxy-phenyl)-carbamatsäure-2-azabicyclo[2.2.1]hept-7-yl-methyl-ester,
3-(2-Aza-bicyclo[2.2.1 ]hept-7-yl-methoxycarbonylamino)-benzoesäure-ethylester,
(4-Fluoro-3-nitro-phenyl)-carbamatsäure-2-benzyl-2-aza-bicyclo[2.2.1]hept-7-yl-methyl-ester,
(3,5-Bis-trifluoromethyl-phenyl)-carbamatsäure-2-benzyl-2-aza-bicyclo[2.2.1]hept-7-yl-methyl-ester,
(3,4,5,-Trimethoxy-phenyl)-carbamatsäure-2-benzyl-2-aza-bicyclo[2.2.1]hept-7-yl-methyl-ester,
(3,4-Dichloro-phenyl)-carbamatsäure-2-benzyl-2-aza-bicyclo[2.2.1]hept-7-yl-methyl-ester,
3-[2-(4-Trifluoromethyl-phenylcarbamoyl)-2-aza-bicyclo[2.2.1]hept-7-yl-methoxycarbonylamino]-benzoesäure-ethylester,
3-[2-(Toluene-4-sulfonyl)-2-aza-bicyclo-[2.2.1]hept-7-yl-methoxycarbonylamino]-benzoesäure-ethylester,
3-(2-Ethoxycarbonylmethyl-2-aza-bicyclo[2.2.1]hept-7-yl-methoxycarbonylamino)-benzoesäure-ethylester,
(3-Cyano-phenyl)-carbamatsäure-2-benzyl-2-aza-bicyclo[2.2.1]hept-7-yl-methyl-ester,
(2,6-Dichloro-pyridin-4-yl)-carbamatsäure-2-benzyl-2-azabicyclo[2.2.1]hept-7-yl-methyl-ester,
(3-Acetyl-phenyl)-carbamatsäure-2-benzyl-2-aza-bicyclo[2.2.1]hept-7-yl-methyl-ester,
1-(2-Benzyl-2-aza-bicyclo[2.2.1]hept-7-ylmethyl)-3-(3,4,5-trimethoxy-phenyl)-harnstoff,
1-(2-Benzyl-2-aza-bicyclo[2.2.1]hept-7-ylmethyl)-3-(2-nitro-phenyl)-harnstoff,
oder ein physiologisch verträgliches Salz davon.

4. Verfahren zur Herstellung einer Verbindung nach einem der Ansprüche 1 bis 3, **dadurch gekennzeichnet, daß** es die Umsetzung einer Verbindung der allgemeinen Formel (II) 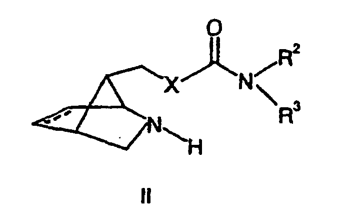 in der X, R² und R³ die in den Ansprüchen 1 oder 2 definierte Bedeutung haben, mit einem Kation R¹⁺ umfaßt, das sich von der Gruppe R¹ ableitet, wobei diese die die in Anspruch 1 oder 2 dargelegte Bedeutung hat und ein geeignetes Gegenion aufweist.

5. Verfahren nach Anspruch 4, **dadurch gekennzeichnet, daß** dieses weiterhin die Umsetzung einer Verbindung der allgemeinen Formel IV 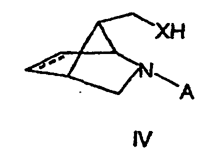 in der X die in den Ansprüchen 1 oder 2 dargelegte Bedeutung hat und A eine geeignete Schutzgruppe, vorzugsweise eine Benzylgruppe darstellt, mit einem Isocyanat der Formel R²-N=C=O, in dem R² die in den Ansprüchen 1 oder 2 dargelegte Bedeutung hat, sowie gegebenenfalls die Abspaltung der Gruppe A, vorzugsweise durch Hydrierung, umfasst.

6. Verfahren nach Anspruch 5, **dadurch gekennzeichnet, daß** die Verbindung der Formel (IV) mit X = NH oder NR⁴ aus der Verbindung mit der Formel (IV), in der X = O ist, hergestellt wird, indem man diese Verbindung in einen geeigneten Sulfonsäureester überführt, diesen mit einem Azid umsetzt und das derart erhaltene Azid in an sich bekannter Weise zu einem primären Amin reduziert, oder den Alkohol mit Stickstoffwasserstoffsäure und einem Azodicarboxylat in Gegenwart eines geeigneten Phosphins, vorzugsweise Triphenylphosphin, zu einem primären Amin umsetzt, sowie das primäre Amin gegebenenfalls auf an sich bekannter Weise in das gewünschte sekundäre Amin mit der in Anspruch 1 gegebenen Bedeutung von R⁴ überführt, vorzugsweise durch Acylierung und nachfolgende Reduktion.

7. Substanz der allgemeinen Formel III 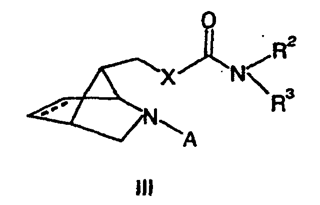 in der X ausgewählt ist aus der Gruppe bestehend NH, NR⁴, O und S,
R² , R³ und R⁴ unabhängig voneinander ausgewählt sind aus der Gruppe bestehend aus C₁-C₁₀-Alkylgruppen, C₅-C₁₂-Arylgrupen und C₄-C₁₂-Heteroarylgruppen, wobei diese Gruppen unsubstituiert sind oder einen oder mehrere Substituenten aufweisen, die unabhängig voneinander ausgewählt sind aus der Gruppe bestehend aus Halogenen, Alkoxygruppen, Aryloxygruppen, Nitrogrupen, C₁-C₅-Alkoxycarbonylgruppen, gegebenenfalls durch Fluor substituierten Alkyl- oder Acylgrupen und Cyanogruppen,
und A Wasserstoff oder eine geeignete Schutzgrupppe, vorzugsweise eine Benzylgruppe ist.

8. Substanz der allgemeinen Formel (IV) 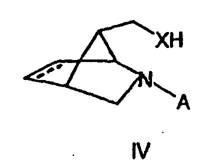 in der A eine geeignete Schutzgruppe, vorzugsweise eine Benzylgruppe, darstelit, und X = NH oder NR⁴ ist, wobei
R⁴ ausgewählt ist aus der Gruppe bestehend aus C₁-C₁₀-Alkylgruppen, C₅-C₁₂-Arylgrupen und C₄-C₁₂-Heteroarylgruppen, wobei diese Gruppen unsubstituiert sind oder einen oder mehrere Substituenten aufweisen, die unabhängig voneinander ausgewählt sind aus der Gruppe bestehend aus Halogenen, Alkoxygruppen, Aryloxygruppen, Nitrogrupen, C₁-C₅-Alkoxycarbonylgruppen, gegebenenfalls durch Fluor substituierten Alkyl- oder Acylgrupen und Cyanogruppen,
und A Wasserstoff oder eine geeignete Schutzgruppe, vorzugsweise eine Benzylgruppe, ist.

9. Arzneimittel, enthaltend eine Verbindung nach einem der Ansprüche 1 bis 3 oder ein physiologisch verträgliches Salz davon neben den üblichen Begleit- und Hilfsstoffen.

10. Verwendung einer Verbindung nach einem der Ansprüche 1 bis 3 oder eines physiologisch verträglichen Salzes davon zur Herstellung eines Arzneimittels zur Behandlung von Krankheiten, bei denen die Anregung der nikotinischen Acetylcholinrezeptoren zur Verbesserung des Krankheitsbildes führt.

11. Verwendung nach Anspruch 10, **dadurch gekennzeichnet, daß** die Krankheiten ausgewählt sind aus der Gruppe bestehend aus Schizophrenie, Demenz, dabei insbesondere Morbus Alzheimer, neurodegenerativen Erkrankungen, Parkinson'scher Krankheit, Tourette's Syndrom, altersbedingte Gedächtnisschwäche, Linderung von Entzugserscheinungen, Schlaganfall und Schädigung des Gehirns durch toxische Verbindungen.

## Claims

1. Substance of the general formula 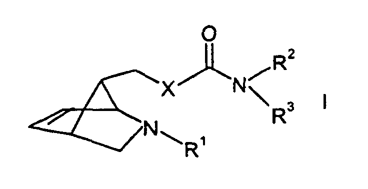 in which
the broken line represents a double bond which is optionally present,
X is selected from the group consisting of NH, NR⁴, O and S,
R¹ is selected from the group consisting of hydrogen, linear and branched, substituted and unsubstituted C₁-C₁₀-alkyl groups, substituted and unsubstituted C₅-C₁₀-aryl groups, substituted and unsubstituted C₄-C₁₀-heteroaryl groups, acyl groups, thioacyl groups, carbonylcarboxy groups, N-organyl-substituted carbamoyl groups and organosulphonyl groups,
R², R³ and R⁴ are selected, independently of one another, from the group consisting of C₁-C₁₀-alkyl groups, C₅-C₁₂-aryl groups and C₄-C₁₂-heteroaryl groups, these groups being unsubstituted or having one or more substituents which are selected, independently of one another, from the group consisting of halogens, alkoxy groups, aryloxy groups, nitro groups, C₁-C₅-alkoxycarbonyl groups, optionally fluorine-substituted alkyl or acyl groups and cyano groups,
or a physiologically tolerated salt thereof.

2. Substance according to Claim 1, in which X is NH, NR⁴ or O, in particular O,
R¹ is selected from the group consisting of hydrogen, linear and branched C₁-C₅-alkyl groups which are unsubstituted or may have as substituents one or more halogen atoms, optionally alkylsubstituted C₆-C₁₀-aryl groups and/or C₁-C₅-alkoxycarbonyl groups, or C₆-C₁₂-aryl groups and C₅-C₁₀-heteroaryl groups which may be unsubstituted or have as substituents in each case one or more halogen atoms and/or alkyl groups, or aliphatic C₁-C₅-acyl and thioacyl groups, aromatic C₆-C₉-aroyl and thioaroyl groups, carbamoyl groups of the type R⁵-N(H)-C(O)- and sulphonyl groups of the type R⁵-SO₂-,
R², R³ and R⁴ are selected, independently of one another, from the group consisting of C₁-C₄-alkyl groups, the C₅-C₉-aryl groups and C₅-C₈-heteroaryl groups which are unsubstituted or have one or more substituents which are selected from the group consisting of C₁-C₄-alkoxy groups, C₆-C₉-aryloxy groups, nitro groups, C₁-C₂-alkoxycarbonyl groups, cyano groups, fluoride, chloride, fluoromethyl groups and acetyl groups.
R⁵ is selected from the group consisting of C₁-C₃-alkyl groups, C₁-C₃-fluoroalkyl groups and phenyl groups which can be unsubstituted or substituted by one or more methyl or trifluoromethyl groups, or a physiologically tolerated salt thereof.

3. 2-Benzyl-2-azabicyclo[2.2.1]hept-7-ylmethyl (2,6-dichlorophenyl)carbamate,
2-Benzyl-2-azabicyclo[2.2.1]hept-7-ylmethyl (4-phenoxyphenyl)carbamate,
2-Benzyl-2-azabicyclo[2.2.1]hept-7-ylmethyl (2-nitrophenyl)carbamate,
Ethyl 3-(2-benzyl-2-azabicyclo[2.2.1]hept-7-ylmethoxycarbonylamino)benzoate,
2-Azabicyclo[2.2.1]hept-7-ylmethyl (3,4-dichlorophenyl)carbamate,
2-Azabicyclo[2.2.1]hept-7-ylmethyl (3,5-bistrifluoromethylphenyl)carbamate,
2-Azabicyclo[2.2.1]hept-7-ylmethyl (2,4-dimethoxyphenyl)carbamate,
Ethyl 3-(2-azabicyclo[2.2.1]hept-7-ylmethoxycarbonylamino)benzoate,
2-Benzyl-2-azabicyclo[2.2.1]hept-7-ylmethyl (4-fluoro-3-nitrophenyl)-carbamate,
2-Benzyl-2-azabicyclo[2.2.1]hept-7-ylmethyl (3,5-bistrifluoromethylphenyl)carbamate,
2-Benzyl-2-azabicyclo[2.2.1]hept-7-ylmethyl (3,4,5-trimethoxyphenyl)carbamate,
2-Benzyl-2-azabicyclo[2.2.1]hept-7-ylmethyl (3,4-dichlorophenyl)-carbamate,
Ethyl 3-[2-(4-trifluoromethylphenylcarbamoyl)-2-azabicyclo[2.2.1]-hept-7-ylmethoxycarbonylamino]benzoate,
Ethyl 3-[2-(toluene-4-sulphonyl)-2-azabicyclo[2.2.1]hept-7-yl-methoxycarbonylamino]benzoate,
Ethyl 3-(2-ethoxycarbonylmethyl-2-azabicyclo[2.2.1]hept-7-yl-methoxycarbonylamino)benzoate,
2-Benzyl-2-azabicyclo[2.2.1]hept-7-ylmethyl (3-cyanophenyl)carbamate,
2-Benzyl-2-azabicyclo[2.2.1]hept-7-ylmethyl (2,6-dichloropyridin-4-yl)carbamate,
2-Benzyl-2-azabicyclo[2.2.1]hept-7-ylmethyl (3-acetylphenyl)carbamate,
1-(2-Benzyl-2-aza-bicyclo[2.2.1]hept-7-ylmethyl)-3-(3,4,5-trimethoxyphenyl)urea,
1-(2-Benzyl-2-aza-bicyclo[2.2.1]hept-7-ylmethyl)-3-(2-nitrophenyl)-urea,
or a physiologically tolerated salt thereof.

4. Process for preparing a compound according to any of Claims 1 to 3, **characterized in that** it comprises reaction of a compound of the general formula (II) 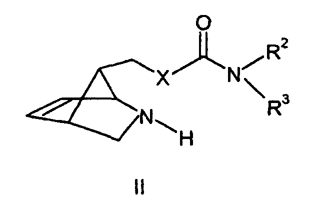 in which X, R² and R³ have the meaning defined in Claims 1 or 2, with a cation R¹⁺ which is derived from the group R¹ where the latter has the meaning set forth in Claim 1 or 2 and has a suitable counter ion.

5. Process according to Claim 4, **characterized in that** it additionally comprises the reaction of a compound of the general formula IV 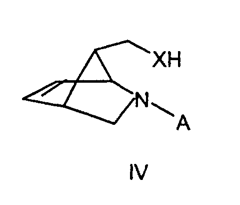 in which X has the meaning set forth in Claims 1 or 2, and A is a suitable protective group, preferably a benzyl group, with an isocyanate of the formula R²-N=C=O, in which R² has the meaning set forth in Claims 1 or 2, and, where appropriate, elimination of the group A, preferably by hydrogenation.

6. Process according to Claim 5, **characterized in that** the compound of the formula (IV) with X = NH or NR⁴ is prepared from the compound with the formula (IV), in which X is O by converting the latter compound into a suitable sulphonic ester, reacting the latter with an azide, and reducing the azide obtained in this way in a manner known per se to a primary amine, or reacting the alcohol with hydrazoic acid and an azodicarboxylate in the presence of a suitable phosphine, preferably triphenylphosphine, to give a primary amine, and converting the primary amine where appropriate in a manner known per se into the required secondary amine with the meaning of R⁴ given in Claim 1, preferably by acylation and subsequent reduction.

7. Substance of the general formula III 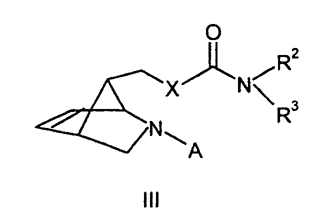 in which X is selected from the group consisting of NH, NR⁴, O and S,
R², R³ and R⁴ are selected, independently of one another, from the group consisting of C₁-C₁₀-alkyl groups, C₅-C₁₂-aryl groups and C₄-C₁₂-heteroaryl groups, these groups being unsubstituted or having one or more substituents which are selected, independently of one another, from the group consisting of halogens, alkoxy groups, aryloxy groups, nitro groups, C₁-C₅-alkoxycarbonyl groups, optionally fluorine-substituted alkyl or acyl groups and cyano groups,
and A is hydrogen or a suitable protective group, preferably a benzyl group.

8. Substance of the general formula (IV) 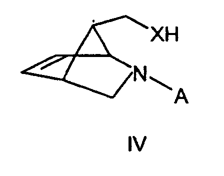 in which A is a suitable protective group, preferably a benzyl group, and X is NH or NR⁴, where
R⁴ is selected from the group consisting of C₁-C₁₀-alkyl groups, C₅-C₁₂-aryl groups and C₄-C₁₂-heteroaryl groups, these groups being unsubstituted or having one or more substituents which are selected, independently of one another, from the group consisting of halogens, alkoxy groups, aryloxy groups, nitro groups, C₁-C₅-alkoxycarbonyl groups, optionally fluorine-substituted alkyl or acyl groups and cyano groups,
and A is hydrogen or a suitable protective group, preferably a benzyl group.

9. Pharmaceutical comprising a compound according to any of Claims 1 to 3 or a physiologically tolerated salt thereof in addition to conventional additional substances and excipients.

10. Use of a compound according to any of Claims 1 to 3 or of physiologically tolerated salt thereof for producing a pharmaceutical for the treatment of diseases in which stimulation of the nicotinic acetylcholine receptors leads to an improvement in the pathological state.

11. Use according to Claim 10, **characterized in that** the diseases are selected from the group consisting of schizophrenia, dementia, in particular Alzheimer's disease, neurodegenerative disorders, Parkinson's disease, Tourette's syndrome, age-related memory weakness, alleviation of withdrawal symptoms, stroke and damage to the brain due to toxic compounds.

## Revendications

1. Substance de la formule générale : 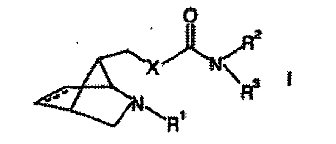 dans laquelle
la ligne en traits pointillés représente une double liaison présente de manière facultative,
X est choisi parmi le groupe consistant en NH, NR⁴, O et S,
R¹ est choisi parmi le groupe consistant en hydrogène, des radicaux alkyle en C₁-C₁₀ linéaires et ramifiés, substitués et non substitués, des radicaux aryle en C₅-C₁₀ substitués et non substitués, des radicaux hétéroaryle en C₄-C₁₀ substitués et non substitués, des radicaux acyle, des radicaux thioacyle, des radicaux carbonylcarboxy, des radicaux carbamoyle N-organyl-substitués et des radicaux organosulfonyle ;
R², R³ et R⁴ sont choisis indépendamment l'un de l'autre, parmi le groupe consistant en des radicaux alkyle en C₁-C₁₀, des radicaux aryle en C₅-C₁₂ et des radicaux hétéroaryle en C₄-C₁₂, où ces radicaux sont non substitués ou présentent un ou plusieurs substituants, qui sont choisis indépendamment l'un de l'autre parmi le groupe consistant en des halogènes, des radicaux alcoxy, des radicaux aryloxy, des radicaux nitro, des radicaux (alcoxy en C₁-C₅)-carbonyle, des radicaux alkyle ou acyle le cas échéant substitués par fluor et le radical cyano,
ou un sel physiologiquement compatible de celle-ci.

2. Substance selon la revendication 1, dans laquelle X = NH, NR⁴ ou O, en particulier O,
R¹ est choisi parmi le groupe consistant en l'hydrogène, des radicaux alkyle en C₁-C₅ linéaires et ramifiés, qui sont non substitués ou peuvent porter comme substituant, un ou plusieurs atomes d'halogène, des radicaux aryle en C₆-C₁₀ et/ou des radicaux (alcoxy en C₁-C₅)carbonyle le cas échéant alkylsubstitués, en des radicaux aryle en C₆-C₁₂ et des radicaux hétéroaryle en C₅-C₁₀, qui sont non substitués ou peuvent porter comme substituant, chaque fois un ou plusieurs atomes d'halogène et/ou radicaux alkyle, des radicaux acyle et thioacyle en C₁-C₅ aliphatiques, des radicaux aroyle et thioaroyle en C₆-C₉ aromatiques, des radicaux carbamoyle de type R⁵-N(H)-C(O)- et des radicaux sulfonyle du type R⁵-SO₂- ;
R², R³ et R⁴ sont choisis indépendamment l'un de l'autre, parmi le groupe consistant en des radicaux alkyle en C₁-C₄, des radicaux aryle en C₅-C₉ et des radicaux hétéroaryle en C₅-C₈, qui sont non substitués ou portent un ou plusieurs substituants, qui sont choisis parmi le groupe consistant en des radicaux alcoxy en C₁-C₄, des radicaux aryloxyen C₆-C₉, des radicaux nitro, des radicaux (alcoxy en C₁-C₂)carbonyle, le radical cyano, des radicaux fluorure, chlorure, fluorométhyle et des radicaux acétyle,
R⁵ est choisi parmi le groupe consistant en des radicaux alkyle en C₁-C₃, des radicaux fluoroalkyle en C₁-C₃ et des radicaux phényle, qui peuvent être non substitués ou substitués par un ou plusieurs radicaux méthyle ou trifluorométhyle,
ou un sel physiologiquement compatible de celle-ci.

3. Ester 2-benzyl-2-azabicyclo[2.2.1]hept-7-yl-méthylique de l'acide (2,6-dichlorophényl)carbamique,
ester 2-benzyl-2-azabicyclo[2.2.1]hept-7-yl-méthylique de l'acide (4-phénoxyphényl)carbamique,
ester 2-benzyl-2-azabicyclo[2.2.1]hept-7-ylméthylique de l'acide (2-nitrophényl)carbamique,
ester éthylique de l'acide 3-(2-benzyl-2-azabicyclo-[2.2.1]hept-7-ylméthoxycarbonylamino)benzoique,
ester 2-azabicyclo[2.2.1]hept-7-ylméthylique de l'acide (3,4-dichlorophényl)carbamique,
ester 2-azabicyclo[2.2.1]hept-7-ylméthylique de l'acide (3,5-bistrifluorométhylphényl)carbamique,
ester 2-azabicyclo[2.2.1]hept-7-ylméthylique de l'acide (2,4-diméthoxyphényl)carbamique,
ester éthylique de l'acide 3-(2-azabicyclo[2.2.1]-hept-7-ylméthoxycarbonylamino)benzoique,
ester 2-benzyl-2-azabicyclo[2.2.1]hept-7-yl-méthylique de l'acide (4-fluoro-3-nitrophényl)carbamique,
ester 2-benzyl-2-azabicyclo[2.2.1]hept-7-yl-méthylique de l'acide (3,5-bistrifluorométhylphényl)-carbamique,
ester 2-benzyl-2-azabicyclo[2.2.1]hept-7-yl-méthylique de l'acide (3,4,5-triméthoxyphényl)carbamique,
ester 2-benzyl-2-azabicyclo[2.2.1]hept-7-yl-méthylique de l'acide (3,4-dichlorophényl)carbamique,
ester éthylique de l'acide 3-[2-(4-trifluorométhylphénylcarbamoyl)-2-azabicyclo[2.2.1]hept -7- ylméthoxycarbonylamino]benzoïque,
ester éthylique de l'acide 3-[2-(toluène-4-sulfonyl)-2-azabicyclo[2.2.1]hept-7-ylméthoxycarbonyl-amino]benzoïque,
ester éthylique de l'acide 3-(2-éthoxycarbonylméthyl-2-azabicyclo[2.2.1]hept-7-ylméthoxycarbonylamino]benzoïque,
ester 2-benzyl-2-azabicyclo[2.2.1]hept-7-yl-méthylique de l'acide (3-cyanophényl)carbamique,
ester 2-benzyl-2-azabicyclo[2.2.1]hept-7-yl-méthylique de l'acide (2,6-dichloropyridin-4-yl)carbamique,
ester 2-benzyl-2-azabicyclo[2.2.1]hept-7-yl-méthylique de l'acide (3-acétylphényl)carbamique,
1-(2-benzyl-2-azabicyclo[2.2.1]hept-7-yl-méthyl)-3-(3,4, 5-triméthoxyphényl)urée,
1-(2-benzyl-2-azabicyclo[2.2.1]hept-7-yl-méthyl)-3 - (2 - nitrophényl)urée,
ou un sel physiologiquement compatible de ceux-ci.

4. Procédé de préparation d'un composé selon une des revendications 1 à 3, **caractérisé en ce qu'**il comprend la réaction d'un composé de la formule générale (II) : 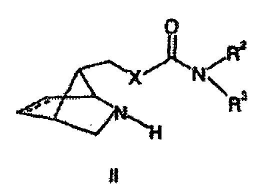 dans laquelle X, R² et R³ ont la signification définie aux revendications 1 ou 2, avec un cation R¹⁺, qui dérive du radical R¹, où celui-ci a la signification définie aux revendications 1 ou 2 et présente un contre-ion approprié.

5. Procédé selon la revendication 4, **caractérisé en ce qu'**il comprend la réaction d'un composé de la formule générale (IV) : 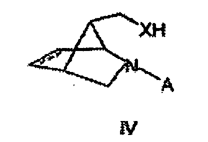 dans laquelle X a la signification définie aux revendications 1 ou 2, et A représente un groupe protecteur approprié, de préférence un radical benzyle, avec un isocyanate de la formule R²-N=C=O, dans laquelle R² a la signification définie aux revendications 1 ou 2, ainsi que le cas échéant, le clivage du radical A, de préférence par hydrogénation.

6. Procédé selon la revendication 5, **caractérisé en ce que** le composé de la formule (IV) avec X = NH ou NR⁴, est préparé à partir du composé de la formule (IV), dans laquelle X est O, **en ce que** l'on convertit ce composé en un ester d'acide sulfonique approprié, on fait réagir celui-ci avec un azoture et l'azoture ainsi obtenu est réduit de manière connue en soi en une amine primaire, ou l'alcool est mis à réagir avec l'acide azothydrique et une azodicarboxylate en présence d'une phosphine appropriée, de préférence la triphénylphosphine, en une amine primaire, et l'amine primaire est le cas échéant convertie, de manière connue en soi, en l'amine secondaire appropriée avec la signification de R⁴ donnée à la revendication 1, de préférence par acylation et ensuite, réduction.

7. Substance de la formule générale III : 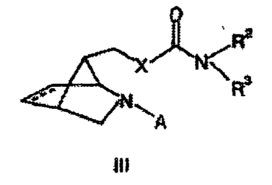 dans laquelle X est choisi parmi le groupe consistant en NH, NR⁴, O et S,
R², R³ et R⁴ sont choisis indépendamment l'un de l'autre, parmi le groupe consistant en des radicaux alkyle en C₁-C₁₀, des radicaux aryle en C₅-C₁₂ et des radicaux hétéroaryle en C₄-C₁₂, où ces radicaux sont non substitués ou présentent un ou plusieurs substituants, qui sont choisis indépendamment l'un de l'autre, parmi le groupe consistant en des halogènes, des radicaux alcoxy, des radicaux aryloxy, le radical nitro, des radicaux (alcoxy en C₁-C₅)carbonyle, des radicaux alkyle ou acétyle le cas échéant substitués par fluor et le radical cyano, et
A est hydrogène ou un groupe protecteur approprié, de préférence le radical benzyle.

8. Substance de la formule générale IV : 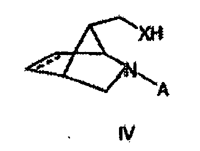 dans laquelle A est un groupe protecteur approprié, de préférence le radical benzyle, et X est NH ou NR⁴, où
R⁴ est choisi parmi le groupe consistant en des radicaux alkyle en C₁-C₁₀, des radicaux aryle en C₅-C₁₂ et des radicaux hétéroaryle en C₄-C₁₂, où ces radicaux sont non substitués ou présentent un ou plusieurs substituants, qui sont choisis indépendamment l'un de l'autre, parmi le groupe consistant en des halogènes, des radicaux alcoxy, des radicaux aryloxy, le radical nitro, des radicaux (alcoxy en C₁-C₅)carbonyle, des radicaux alkyle ou acétyle le cas échéant substitués par fluor et le radical cyano, et
A est hydrogène ou un groupe protecteur approprié, de préférence le radical benzyle.

9. Agent pharmaceutique, contenant un composé selon l'une quelconque des revendications 1 à 3 ou un sel physiologiquement compatible de celui-ci, avec les additifs et auxiliaires usuels.

10. Utilisation d'un composé selon l'une quelconque des revendications 1 à 3 ou un sel physiologiquement compatible de celui-ci, pour la préparation d'un agent pharmaceutique pour le traitement de maladies, pour lesquelles l'activation des récepteurs nicotiniques de l'acétyl-choline conduit à l'amélioration de la maladie.

11. Utilisation selon la revendication 10, **caractérisée en ce que** les maladies sont choisies parmi le groupe consistant en la schizophrénie, la démence, en particulier la maladie d'Alzheimer, les maladies neuro-dégénérative, la maladie de Parkinson, le syndrome de Tourette, les troubles de la mémoire liés à l'âge, le soulagement du syndrome de manque, l'attaque et les troubles cérébraux par des composés toxiques.
